Europäisches Patentamt

(19) European Patent Office

Office européen des brevets

(11) EP 0 888 786 A2

(12) **EUROPEAN PATENT APPLICATION**

(43) Date of publication:
07.01.1999 Bulletin 1999/01

(51) Int. Cl.⁶: **A61L 15/58**

(21) Application number: 98112060.3

(22) Date of filing: 30.06.1998

(84) Designated Contracting States:
AT BE CH CY DE DK ES FI FR GB GR IE IT LI LU MC NL PT SE
Designated Extension States:
AL LT LV MK RO SI

(30) Priority: 02.07.1997 US 887446

(71) Applicant:
National Starch and Chemical Investment Holding Corporation
Wilmington, Delaware 19803-7663 (US)

(72) Inventors:
• Tong, Quinn K.
Belle Mead, N.J. 08502 (US)
• Sharak, Matthew L.
Franklin Park, N.J. 08823 (US)
• Wu, Bing
Marina del Rey, California 90292 (US)
• Riswick, Martin W.
Marlow, Bucks SL7 1SQ (GB)
• Khan, Rosina
Slough, Berkshire SL1 2QT (GB)

(74) Representative:
Held, Stephan, Dr.rer.nat., Dipl.-Chem. et al
Patentanwälte,
Hagemann, Braun und Held,
Patentanwälte,
Postfach 86 03 29
81630 München (DE)

(54) **Absorbent articles comprising a polyether-containing hot melt adhesive, and hot melt adhesive compositions comprising polyethers in combination with surfactants**

(57) The subject invention provides absorbent articles which comprise hot melt adhesive compositions which comprise an adhesive base polymer and a polyether, and which impart improved wicking capabilities to the articles. Also provided is a hot melt adhesive composition comprising a base polymer, a mixture of one or more polyethers, and one or more surfactants.

EP 0 888 786 A2

Printed by Xerox (UK) Business Services
2.16.5/3.4

## Description

This invention relates to hot melt adhesives which are used in absorbent articles such as diapers, sanitary napkins, incontinence guards, and the like.

In absorbent articles, such as diapers, sanitary napkins, and incontinence guards, it is beneficial if each of the components comprising the article contributes to the overall fluid absorption capability of the article. This is especially true when one wishes as an objective to create as thin of an absorbent article as possible.

Absorbent articles typically comprise multiple layers, one or more of which is typically a nonwoven fabric layer. A nonwoven fabric (a "nonwoven") is an interlocking network of synthetic and/or naturally occurring fibers, in which the individual fibers are mechanically, chemically, and/or thermally bonded to each other. Tissue is a nonwoven fabric comprising individual natural fibers mechanically bonded to one another. In addition to the absorbent articles mentioned above, nonwoven fabric is used commercially in a variety of applications including insulation, packaging, household wipes, surgical drapes, and medical dressings.

In absorbent articles, as well as in other applications of nonwovens, it is often necessary to adhere a nonwoven component, such as a layer, to another component of the article. The second component may also be made of another nonwoven, or may be made of a different material, such as a polyethylene film. Hot melt adhesives are commonly used to adhere the components of an absorbent article together. Hot melt adhesives are also commonly used to bond together the fibers of a nonwoven in an absorbent article.

A desired property for hot melt adhesives which are to be used in absorbent articles is an ability to transmit fluids away from the body, through the layers of the article, and into the core of the article. This property is referred to as "wicking". "Strike through time", or simply "strike through", is the time required for a liquid to reach the core of an article or substrate from the surface, and is a way of evaluating wicking.

When a droplet of an aqueous liquid is placed on a uniform, perfectly flat, solid surface, its shape and the length of time that it holds it shape are determined by three interfacial tension forces: the force of the solid surface, the surface tension of the liquid, and the force at the solid/liquid interface. The angle that the tangent of the liquid droplet makes with the solid surface (as measured from the solid/liquid interface) is a product of these three forces. In general, the smaller this contact angle, $\Theta$, the greater the tendency of the liquid droplet to lose its shape and spread. The flow of fluid through a nonwoven can be modeled as flow through a series of capillaries and thus the Lucas-Washburn equations can be applied. These equations indicate that both the wicking rate and the wicking height are reduced when the contact angle $\Theta$ is large (cos 90 = 0!):

$$\text{Wicking Rate } \frac{dh}{dt} = \frac{1}{2} \left[ \frac{r\sigma \cos\Theta}{2\eta t} \right]^{\frac{1}{2}} \quad h \ll H$$

$$\text{Ultimate Wicking Height } H = \frac{2\sigma \cos\Theta}{\_gr} ;$$

where h is the current wicked height at time t, r is an effective capillary radius, $\sigma$ is the fluid surface tension, $\eta$ is the fluid viscosity, _ is the fluid density, g is the gravitational constant, and $\Theta$ is the contact angle the liquid makes with the capillary wall.

The addition of surfactant to a hot melt adhesive causes a decrease in the contact angle $\Theta$. As a result, the surfactant makes the hot melt adhesive more effective in transmitting fluids through the adhesive layer or through any layer comprising the adhesive. However, the intermingling of the surfactant with the fluid may also cause a reduction of surface tension in the fluid droplets, lessening the capability of the fluid to wick through to the core of the article. Thus, in preparing hot melt adhesive compositions comprising surfactant for use in absorbent articles, a balance must be struck between fluid surface tension reduction (STR) and decreasing the fluid contact angle to achieve an acceptable strike through.

U.S. Serial No. 08/631,390, filed April 12, 1996, describes a hot melt adhesive composition containing 1 to 25% of a surfactant, which causes the adhesive composition to exhibit a contact angle of 90 degrees or less and a reduction in surface tension of less than or equal to about 35-40 dyne/cm, giving improved wicking capabilities to a nonwoven disposable article. U.S. Serial No.08/631,390 does not describe or suggest adding polyethers, such as polypropyleneglycol (PPG), which have no surfactant activity, to a hot melt adhesive.

GB 2 294 397 A, Date of A Publication May 1, 1996, relates to an adhesive used in an absorbent article, which adhesive contains a surfactant, preferably a non-ionic surfactant such as an alcohol, alkanolamide, amino oxide, ester, or ether. GB 2 294 397 does not indicate using any substance such as PPG.

U.S. Patent No. 5,478,335, issued December 26, 1995 to Colbert, describes an absorbent device which comprises a cover sheet comprising an apertured polymer film and an intermediate layer comprising a planar apertured polymer film. At least one of the apertured film layers may contain a surfactant. U.S. Patent No. 5,478,335 does not, however, mention using PPG in the device, nor is a polyether suggested.

It is also known to use fluorochemical surfactants in some hot melt adhesives to improve strike through without reducing the absorbency speed or capacity of the absorbing material (see, for example, EP 0 710 737 A2, Date of Publication May 8, 1996). However, fluorosurfactants are costly, absorb through skin, and are relatively toxic.

Thus, a need exists for novel hot melt adhesive compositions which have improved wicking performance. Conventional hot melt adhesives, without added surfactant, have good cytotoxicity results. However, the base polymer in such hydrophobic hot melt adhesives appears to act as a barrier, hindering the liquid penetration in nonwoven applications, such as (but not limited to) diapers and sanitary napkins. Some hot melt adhesive compositions described in the prior art used relatively high levels of surfactants to achieve low water contact angle and fast liquid strike through time. The subject invention offers an alternative means for reducing $\Theta$.

We have discovered that, by adding polyethers such as PPG, which have a variety of molecular weights and which are compatible with hydrophobic hot melt materials, to hot melt adhesive compositions, the wicking performance of the hot melt adhesive improves significantly. Using polyethers, optionally with other surfactants, in the hot melt formulation, significantly decreases the liquid contact angle $\Theta$ of the adhesive. Polyethers do not significantly decrease the surface tension of the liquid. This low contact angle $\Theta$ and low surface tension reduction indicate improved wicking performance of the adhesive. We determined, as shown by the experiments described later on in this application, that hot melt adhesive compositions which comprise polyether compounds possess significantly improved liquid strike through time and repeat strike through time, yet maintain good adhesive bond strength. Additionally, polyethers can be combined with surfactants to reduce $\Theta$ to minimize surfactant leaching from the hot melt adhesive composition, and any toxicity concern of surfactant which has leached from an absorbent article also improves dramatically. Moreover, all of the hot melt adhesive compositions comprising polyethers exemplary of our invention which we tested, some of which further comprised low levels of surfactants, remained stable at high temperature over time. Our invention thus involves novel hot melt adhesive compositions which have improved water wicking performance and improved strike through time.

This invention provides absorbent articles possessing improved wicking ability, and novel hot melt adhesive compositions useful for obtaining such a result. In particular, this invention provides an absorbent article comprising a hot melt adhesive composition, which composition comprises a) a base polymer in an amount effective to render the composition adhesive, and b) a mixture of one or more polyethers in an amount effective to cause a fluid contacting the article to exhibit a fluid contact angle or less than about 90 degrees and a fluid surface tension reduction of less than about 40 dyne/cm, said polyethers consisting essentially of homo, alternating, or random units having the formula

$$-R-O-,$$

wherein each R is selected from the group consisting of alkyl moieties having 2 or more carbon atoms. Furthermore, the incorporation of a polyether or polyethers into the hot melt adhesive composition improves the repeat strike through time of the article.

The term "fluid contact angle" refers to the angle that the tangent of a droplet of fluid makes with the surface of the absorbent article. The fluid contact angle is sometimes referred to as "$\Theta$". In general, the smaller the fluid contact angle, the greater the tendency of the fluid droplet to lose its shape and spread. Throughout this application, the term "liquid" is used interchangeably with the term "fluid". For example, "liquid contact angle" means the same thing as "fluid contact angle".

Fluid surface tension reduction, or "STR", means the difference between the surface tension of a pure fluid and the surface tension of the fluid after exposure to the hot melt adhesive composition. Generally, with hot melt adhesives which are incorporated into absorbent articles, it is desirable to have a hot melt adhesive composition which does not cause a high surface tension reduction in a fluid, as reduction in the fluid's surface tension decreases the ability of the fluid to wick through the absorbent article.

The phrase "repeat strike through time" refers to the time required for a liquid to wick through a substrate, which substrate has previously been exposed to said liquid.

For purposes of this invention, an "absorbent article" is any article which is used to absorb a liquid. Absorbent articles include, but are not limited to, diapers, sanitary napkins, and incontinence guards. Liquids which may be absorbed by absorbent articles according to this invention may be any liquid, including aqueous liquids, such as water, urine, and blood.

In one embodiment, the absorbent articles of this invention are comprised at least in part of a nonwoven fabric. A nonwoven fabric (or "nonwoven") is an interlocking network of synthetic and/or naturally occurring fibers, in which the individual fibers are mechanically, chemically, and/or thermally bonded, rather than woven, together. For example, the fibers of a nonwoven fabric may be mechanically, or mechanically and thermally, bonded together by means of a press. In one embodiment of this invention, the fibers of the nonwoven component of an absorbent article are bonded together by the hot melt adhesive composition of this invention.

The subject invention provides absorbent articles which are comprised in some way of the hot melt adhesive compositions described herein. In one embodiment of an absorbent article according to this invention, a hot melt adhesive composition as described herein bonds components of the article together. The adhesive composition may bond several different components of the article together, for example several different layers of the article. Or, the composition may bond just two components together. The components may be any components used to form the article, and said components may be made of any material used to make components of absorbent articles. The components may, for example be made of a nonwoven fabric, or a bonded component may be a polyethylene film. If an absorbent article comprises one or more components which are comprised of a nonwoven fabric, this invention also allows for the subject hot melt adhesive composition to bond together fibers of the nonwoven fabric of such components.

Sources for polyethers for use in the subject hot melt adhesive compositions are known to those of ordinary skill in the art. For example, polypropyleneglycol may be obtained from Arco or Bayer. Polyethers may also be synthesized using any method known in the art.

The polyethers of this invention consist essentially of homo, alternating, or random -R-O- units. For this invention "consisting essentially of" means that there may be one or more units in a polyether which is not a -R-O-as defined above, so long as the properties of such a polyether are not significantly changed. The term "homo" means that the R of each -R-O-unit is the same for all -R-O- units in the polymer. The term "alternating" means that the polyether comprises two or more different alkyl groups for R, and that the -R-O- units having each different type alternate with one another. In one embodiment, the polyether comprises two different types of units which alternate. In another embodiment, the polyether comprises three different types of units which alternate with one another. The term "random" means that the polyether has two or more different alkyl groups for R, and that the units containing these different R groups are randomly dispersed in no predictable pattern within the polymer.

The alkyl moieties for R comprise 2 or more carbon atoms. Preferably, the alkyl moieties have from 2 to 5 carbon atoms, for example 3 carbon atoms or 4 carbon atoms. The alkyl moieties for R may be saturated or unsaturated, or cyclic or acyclic. R may be straight or branched, aromatic or aliphatic. Examples of polyethers which may be used for the subject invention include, but are not limited to, PPG and polytetrahydrofuran. The subject invention may comprise a single polyether (i.e. a "mixture" of one polyether) or a combination of polyethers (i.e. a "mixture" or more than one polyethers).

Polyethers useful for the subject invention are of various molecular weights. Preferably, polyethers which are used for the invention have a molecular weight of 425 Daltons or higher, a molecular weight of between about 2000 Daltons and about 4000 Daltons being more preferred.

If PPG is used as a polyether for this invention, the PPG preferably has a molecular weight between about 2000 and about 4000 Daltons. In one embodiment, the PPG of the subject hot melt adhesive composition has a molecular weight of about 3000.

The amount of polyether used in the subject adhesive compositions is that amount which will cause a fluid contact angle of less than about 90 and a fluid surface tension reduction of less than about 40 dyne/cm, and one of ordinary skill in the art can determine a suitable amount of polyether accordingly, by testing the fluid contact angle and surface tension reduction of a composition comprising polyether using known techniques. In one embodiment the fluid contact angle is less than about 70 degrees, more particularly less than about 50 degrees. In a further embodiment the fluid contact angle is less than about 25 degrees.

In another embodiment, the polyether is added in an amount such that the surface tension reduction is less than about 30 dyne/cm, most preferably less than about 20 dyne/cm.

We have also discovered as part of this invention that combining a surfactant with polyethers as defined above in a hot melt adhesive composition further improves the repeat strike through time and fluid wicking abilities of absorbent articles comprising such adhesive compositions. Thus, this invention also provides an absorbent article as described above, however wherein the hot melt adhesive composition of the article further comprises a surfactant or surfactants in an amount effective in combination with the amount of the mixture of one or more polyethers to cause a fluid contacting the article to exhibit a fluid contact angle of less than about 90 degrees and a fluid surface tension reduction of less than about 40 dyne/cm. This hot melt adhesive composition comprising the surfactant or surfactants along with the mixture of one or more polyethers also falls within the scope of the subject invention. In one embodiment the fluid contact angle is less than about 50 degrees. In a further embodiment the fluid contact angle is less than about 25 degrees.

The surfactants used for this invention are any surfactants which in combination with PPG improve the wicking capabilities of an article comprised of the adhesive composition such that a fluid contacting the article exhibits a fluid contact angle of less than about 90 degrees and a fluid surface tension reduction of less than about 40 dyne/cm. Suitable surfactants may thus be determined by those of ordinary skill.

For example, crystalizable type surfactants, such as Unithox 480 Ethoxylate, a product of Petrolite Specialty Polymers Group, Tulsa, Oklahoma, may be used in the hot melt adhesive compositions of this invention.

Also, nonionic surfactants, including silicone surfactants and linear alcohols (like Unithox 480 Ethoxylate, mentioned above); anionic surfactants; and cationic surfactants may be used in this invention.

Without limiting the scope of the invention, the following nonionic surfactants may be used in the subject hot melt adhesive compositions:

ethoxylates of (i) $C_1$-$C_{18}$, especially $C_8$-$C_9$ alkyl or dialkyl phenols, such as those sold under the tradenames Macol DNP-10, available from PPG industries, Gurnee, Illinois, a 10 mole ethoxylate of dinonyl phenol, and Triton X-100, available from Union Carbide, a 10 mole ethoxylate of octyl phenol; (ii) alkyl $C_8$-$C_{60}$ monoalcohols, such as those sold under the tradenames Surfonic L-12-8, an 8 mole ethoxylate of dodecanol, available from Huntsman Chemical Co., and Unithox 480 Ethoxylate, an ethoxylated $C_{30}$ mono-ol with a molecular weight of 2250 g/mol, available from Petrolite Specialty Polymers Group, Tulsa, Oklahoma; and (iii) propylene oxide polymers, such as those sold under the tradename Pluronic, which are ethylene oxide/propylene oxide block copolymers having a Mn of 200 to 3000 available from BASF; and
benzoates formed by partial condensation of benzoic acid with hydrophilic di or mono-ols having less than 1000 MW, such as the product of condensing about three equivalents of benzoic acid with four equivalents of diethylene glycol, commercially available as XP 1010 from Velsicol Chemical.

Nonlimiting examples of suitable anionic surfactants are:

$C_8$-$C_{60}$ alkyl ethoxylate sulfonates, $(CH_3$-$(CH_2)_{11\text{-}14}$-$(O$-$CH_2CH_2)_3$-$SO^{3\text{-}}Na^+$, such as Avenel S30, available from PPG Industries;

alkyl $C_8$-$C_{60}$ sulfonates, such as Rhodapon UB ($C_{12}$-$SO_3^-Na^+$) available from Rhone Poulenc;
dialkyl $C_4$-$C_{60}$ sulfosuccinates, such as di(2-ethylhexyl)sulfosuccinate available from Cytec Inc. under the tradename Aerosol 07-100;
and alkyl/aromatic sulfonates, such as dodecyl benzene sodium sulfonate, sold under the tradename Calsoft.

Examples of suitable silicone surfactants include, but are not limited to, ethoxylates or propoxylates of polydimethyl siloxane, having a number average molecular weight of 500 to 10,000, preferably 600 to 6000, such as are sold under the tradenames Silwet L-77, L-7605, L-7500, and Nuwet 550 available from OSi Specialties, Danbury, Connecticut; and product 193 from Dow Corning.

Also, polyether/surfactant blends such as Nuwet 500 may be used for the subject hot melt adhesive compositions, serving to provide both ingredients (b) and (c) of the compositions. If such blends are used, the hot melt adhesive compositions may comprise an additional polyether or polyethers and/or an additional surfactant or surfactants to reduce the liquid contact angle $\Theta$.

The amount of surfactant used in the subject adhesive compositions is that amount which in combination with the mixture of polyethers in a composition will cause a fluid contact angle of less than about 90 and a fluid surface tension reduction of less than about 40 dyne/cm, and one of ordinary skill in the art can determine a suitable amount of surfactant accordingly, by testing the fluid contact angle and surface tension reduction of a composition using known techniques. In one embodiment the fluid contact angle is less than about 70 degrees, more particularly, less than about 50 degrees. In a further embodiment the fluid contact angle is less than about 25 degrees.

In another embodiment, the surfactant or surfactants is added in an amount which in combination with the mixture of polyethers such that the surface tension reduction is less than about 30 dyne/cm, most preferably, less than about 20 dyne/cm.

The base polymers which are used in the subject hot melt adhesive compositions, either with or without added surfactant(s), are any of the polymers which are used in hot melt adhesives, and such polymers are well known to those of ordinary skill in the art. In one embodiment of this invention, the base polymer is an olefin containing polymer. Polymers which are suitable include olefin containing polymers such as polymers in which ethylene is polymerized with 15 to 45% by weight of copolymerizable monomers such as vinyl acetate, N-butyl acrylate, propylene, methyl acrylate, methyl acrylic acid, acrylic acid, metallocene catalyzed ethylene based polymers and the like, as well as any mixtures thereof. Other polymers which are suitable as the base polymer for the subject hot melt adhesive compositions are pure homopolymers or copolymers of the following monomers: olefins, such as ethylene, propylene, butene, hexene octene, and other alpha-olefins; vinyl monomers, such as vinyl acetate, vinyl propionate, vinyl butyrate, vinyl hexanoate; acrylic monomers, such as acrylic acid and methacrylic acid, methacrylic acid esters, hydroxy ethyl acrylate, and the like.

Preferred for use as the base polymer in the subject compositions are ethylene/vinyl acetate copolymers such as those obtainable from Dupont under the Elvax tradename. The preferred range for the vinyl acetate will be in the range of 18% - 40% by weight, with 28% and 33% being most preferred.

Also preferred as the base polymer are polyolefin polymers such as those obtainable under the Vestoplast tradename from Huls.

Other polymers which may be used as a base polymer for the adhesive compositions of the subject invention are amorphous polyolefins or blends thereof. Amorphous polyolefins are made by the atactic polymerization of polypropylene. Polymerization occurs in the presence of a catalyst comprising a coordination complex of a transition metal halide with an organometallic compound. The solid amorphous polypropylene has a softening point of about 150 degrees Celsius and a Brookfield viscosity at 190 degrees Celsius of 1,000 to 50,000 cps. Suitable commercial products include Eastman Chemical's P 1010. Copolymers of amorphous polypropylene and ethylene (APE), or butene (APB), or hexene (APH), are suitable as a base polymer, as are terpolymers of propylene, butene and ethylene (APBE). Suitable commercially available products include those sold under the tradenames Rextac 2315 from Rexene (APE), Rextac 2730 from Rexene (APB), Vestoplast 750 and 708 from Huls (APBE).

Blends, such as blends of any of the above polymers, may also be used as a base for the hot melt adhesive compositions of this invention. For example, blends of ethylene vinyl acetate and atactic polypropylene may be used to prepare a base for the compositions of this invention.

The amount of base polymer used in the subject hot melt adhesive composition is any amount which will render the composition adhesive, and methods for determining such amounts are well known in the art.

The adhesive compositions used for the subject invention may also optionally comprise tackifying resins, plasticizers, stabilizers, such as antioxidants, waxes and/or other conventional hot melt adhesive additives in varying amounts known to those of ordinary skill in the art. Tackifying resins, plasticizers, stabilizers, waxes and other hot melt adhesive additives are well known in the art, and any such additives may be included in the subject hot melt adhesive compositions.

For example, any compatible hydrocarbon resin, synthetic polyterpene, rosin ester, natural terpene, or the like may be used as a tackifying resin in the compositions of this invention. A suitable tackifying resin for use in any particular composition according to this invention may depend on the particular base polymer of the composition, and selecting a suitable tackifying resin based on the base polymer is well known in the art. Examples of substances which may be suitable as a tackifying resin for a composition according to this invention include, but are not limited to: (1) natural and modified rosins, for example, gum rosin, wood rosin, tall oil rosin, distilled rosin, hydrogenated rosin, dimerized rosin, and polymerized rosin; (2) glycerol and pentaerythritol esters of natural and modified rosins, for example, the glycerol ester of pale wood rosin, the glycerol ester of hydrogenated rosin, the glycerol ester of polymerized rosin, the pentaerythritol ester of hydrogenated rosin, and the phenolic-modified pentaerythritol ester of rosin; (3) copolymers and terpolymers of natural terpenes, for example, styrene/terpene and alpha methyl styrene/terpene; (4) polyterpene resins having a softening point, as determine by ASTM method E28-58T, of 80 degrees to 150 degrees Celsius, the latter polyterpene resins generally resulting from the polymerization of terpene hydrocarbons such as bicyclic monoterpene known as pinene, the polymerization occurring in the presence of Friedel-Crafts catalysts at moderately low temperatures; and hydrogenated polyterpene resins; (5) phenolic modified terpene resins and hydrogenated derivatives thereof, for example, the resin product resulting from the condensation in acidic medium of a bicyclic terpene and a phenol; (6) aliphatic petroleum hydrocarbon resins having a Ball and Ring softening point of 70 degrees to 135 degrees Celsius; the latter resins resulting from the polymerization of monomers primarily consisting of olefins and di-olefins; and the hydrogenated aliphatic petroleum hydrocarbon resins; (7) aromatic petroleum hydrocarbon resins and hydrogenated derivatives thereof; (8) alicyclic petroleum hydrocarbon resins and hydrogenated derivatives thereof; (9) aromatic/aliphatic or alicyclic hydrocarbon resins, such as those sold under the trademarks ECR 149B and ECR 179A by Exxon Chemical Company. One or more than one suitable tackifying resin substance may be used in the adhesive compositions of the subject invention.

Various plasticizing or extending oils may also be present in the compositions of this invention. Suitable amounts of such plasticizing or extending oils may be determined using methods known to those of ordinary skill in the art. In one embodiment of the subject invention, the hot melt adhesive compositions contain up to about 20%, preferably 0 to about 15%, by weight of a plasticising or extending oil in order to provide wetting action and/or viscosity control. In addition to the more usual plasticizing oils known in the art, olefin oligomers and low molecular weight polymers, as well as vegetable and animal oils, and derivatives of animal and vegetable oils, may be used as plasticizing oils in the subject compositions. Petroleum oils that may be employed in the subject compositions include relatively high boiling materials containing only a minor proportion of aromatic hydrocarbons (preferable less than 30%, and, more preferably, less than 15% by weight of the oil). Also suitable are oils which are totally non-aromatic. Oligomers suitable for a plasticizer or extending oil include, but are not limited to, polypropylene oligomers, polybutene oligomers, hydrogenated polyisoprene, hydrogenated polybutadiene, and the like, having an average molecular weight of between about 350 and about 10,000. Vegetable and animal oils which are suitable as a plasticizer or extending oil include, but are not limited to, glyceryl esters of the usual fatty acids and polymerization products thereof.

Polar synthetic compounds, such as the aliphatic and aromatic polyester plasticizers available from C.P. Hall Co., Stow, Ohio, are also useful as plasticizers in the subject hot melt adhesive compositions. Amides phosphate esters, sulfonamides, and phthalates are also suitable at varying levels.

Various waxes, such as certain petroleum derived waxes, may optionally be employed in the compositions of the subject invention in order to impart fluidity in the molten condition of the compositions and flexibility to the compositions once they have set. Such waxes also serve as a wetting agent for bonding cellulosic fibers. For purposes of the subject invention, "petroleum derived wax" includes both paraffin and microcrystalline waxes having melting points within the range of 130 degrees to 225 degrees Fahrenheit, as well as synthetic waxes such as low molecular weight polyethylene or Fisher-Tropsch waxes.

A stabilizer, such as an antioxidant, may also optionally be included in the adhesive compositions of this invention. In one embodiment, a stabilizer is included in the subject hot melt adhesive composition in an amount of up to about 3% by weight. Among the applicable stabilizers are high molecular weight hindered phenols and multifunctional phenols, such as sulfur and phosphorous-containing phenols. Representative hindered phenols include: 1,3,5-trimethyl 2,4,6-tris(3,5-di-tert-butyl-4-hydroxy-benzyl)benzene; pentaerythritol tetrakis-3(3,5-di-tert-butyl-4-hydroxyphenyl)-propionate; n-octadecyl-(3,5-di-tert-butyl-4-hydroxyphenol)-propionate; 4,4'methylenebis(2,6-tert-butylphenol); 4,4'-thiobis (6-tert-butyl-o-cresol); 2,6-di-tertbutylphenol; 6-(4-hydroxyphenoxy)-2,4-bis(n-octyl-thio)-1,3,5-triazine; di-n-octadecyl 3,5-di-tert-butyl-4-hydroxy-benzyl-phosphonate; 2-(n-octylthio)-ethyl 3,5-di-tert-butyl-4-hydroxybenzoate; and sorbitol hexa[3-(3,5-di-tert-butyl-4-hydroxyphenyl)-propionate].

Other additives conventionally used in hot melt adhesives to satisfy different properties and meet specific application requirements also may be added to the subject compositions. Examples of other additives conventionally used in hot melt adhesive compositions include, but are not limited to, fillers, pigments, flow modifiers, and dyestuffs.

In one embodiment, the absorbent articles of this invention comprise a hot melt adhesive which comprises from about 10 to about 80 percent by weight of the base polymer (a), more particularly from about 40 to about 60 percent by weight; and from about 1 to about 25 percent by weight of the mixture (b), more particularly from about 3 to about 10 percent by weight. If the hot melt adhesive composition comprises (c), a surfactant or surfactants, in one embodiment the composition comprises from about 0.5 to about 10 percent by weight, more particularly from about 1 to about 5 percent by weight of (c). The hot melt adhesive composition of this invention in another embodiment comprises from about 10 to about 70 percent by weight of a tackifier in addition to the surfactant or surfactants.

This invention also provides a hot melt adhesive composition comprising from about 10 to about 80 percent by weight of an ethylene/vinyl acetate copolymer, from about 3 to about 10 percent by weight of polypropyleneglycol, and from about 0.5 to about 10 percent by weight of Nuwet 500 or Nuwet 550. Also, this invention provides a hot melt adhesive composition comprising from about 10 to about 80 percent by weight of an ethylene/vinyl acetate copolymer and from about 3 to about 10 percent by weight of Nuwet 500.

The subject hot melt adhesive compositions may be prepared using techniques known to those of ordinary skill in the art. The compositions may, for example, be prepared by blending the components in a melt at a temperature of about 100 to 200 degrees Celsius until a homogenous blend is obtained, generally for about two hours. Various methods of blending are known and any method that produces a homogenous blend is satisfactory. The compositions of this invention so prepared are characterized in that they have a viscosity of 50,000 cP or less at the application temperature of 350 degrees Fahrenheit (177 degrees Celsius) or less. The viscosity as used herein is a Brookfield viscosity measured using a Brookfield viscometer model No. DV-II with spindle no. 27 at 10 rpm.

The adhesive compositions of this invention are also characterized by their ability to provide a durable bond to a nonwoven and otherwise meet the unique requirements of application as adhesives in absorbent articles (including flexibility, non-staining, and machinable viscosity). The adhesive compositions of this invention also possess exceptional thermal stability, which distinguishes them from other moisture sensitive technologies. Furthermore, their hydrophilic character facilitates ready transmission of fluid through the layers and into the structural core of an absorbent article.

The adhesive compositions of the subject invention can be applied to a substrate such as a nonwoven component of an absorbent article by means of a variety of methods including coating or spraying an amount of adhesive composition sufficient to cause the component to adhere to another component.

The subject invention also provides a method for decreasing the fluid contact angle of an absorbent article comprising a hot melt adhesive composition, which method comprises including a mixture of one or more polyethers in said hot melt adhesive composition prior to manufacturing said absorbent article; the polyethers of said mixture consisting essentially of homo, alternating, or random units having the formula

-R-O-,

wherein R is an alkyl moiety having 2 or more carbon atoms.

This invention further provides a method for decreasing the fluid contact angle of an absorbent article comprising a hot melt adhesive composition, which method comprises including in said hot melt adhesive composition prior to manufacturing the absorbent article: a) a mixture of one or more polyethers which consist essentially of homo, alternating, or random units having the formula -R-O-, wherein R is an alkyl moiety having 2 or more carbon atoms; and b) one or more surfactants.

The following examples are merely provided to help illustrate the subject invention, and are not intended to, and should not in any way be construed to, limit the subject invention as defined in the claims of this application.

**Examples**

Sample hot melt adhesive compositions were prepared and tested for water contact angle ($\Theta$), surface tension reduction (STR), viscosity, and heat stability. The following raw materials were used in preparing the hot melt adhesive compositions:

Polypropyleneglycol Arcol 3025, PPG obtained from Arco, number indicates approximate molecular weight;

Elvax 140W, an ethylene/vinyl acetate (EVA) copolymer from DuPont containing 33% vinyl acetate (VA), having a melt flow index (MFI) of 400 (dg/min at 190 degrees Celsius using a 2.2 kg weight);

Elvax 210, an EVA copolymer from DuPont containing 28% VA, having a MFI of 400;

Zonatac 105L, a styrenated terpene from Arizona Chemical with a softening point of 105 degrees Celsius;

Irganox 1010, a hindered phenol anti-oxidant available from Ciba-Geigy;

XR5108, a styrenated terpene tackifier with a 90 degree Celsius softening point available from Arizona Chemical;

Unithox 480 Ethoxylate, an ethoxylated $C_{30}$ mono-ol with a molecular weight of 2250 g/mol available from Petrolite Specialty Polymers Group;

Nuwet 500, a hydrophilic blend of >65% organomodified polydimethyl siloxane, <20% polyalkylene oxide, and <20% ethoxylated alkyl;

Nuwet 550, a polyalkyleneoxide-modified polydimethylsiloxane available from Osi Specialties;

Hercolite 240, an alpha-methyl styrene/styrene tackifier with a 120 degree Celsius softening point available from Hercules;

Eastotac H100, a dicyclopentadiene-based tackifier with a 100 degree Celsius softening point available from Eastman Chemical Co.;

ECR-179A, an aromatic modified dicyclopentadiene tackifier with a 102 degree Celsius softening point available from Exxon;

ECR 179 EX, an aromatic modified dicyclopentadiene tackifier with a 102 degree Celsius softening point available from Exxon;

ECR 5400, a dicyclopentadiene tackifier with a 100 degree Celsius softening point available from Exxon;

Sylvatac 1100, a rosin ester of pentaerythritol tackifier with a softening point of 100 degrees Celsius, available from Arizona Chemical;

Unitac R-98L, hydrogenated rosin ester available from Union Camp;

Rextac RT2730, amorphous poly(alpha olefin) with a 107 degree Celsius softening point from Rexene;

Wingtack Extra, styrenated aliphatic tackifier with a 102 degree Celsius softening point from Goodyear;

Eastotac H130, a dicyclopentadiene-based tackifier with a 130 degree Celsius softening point available from Eastman Chemical Co.;

XW23.AH, Escorene XW23.AX, ethylene n-butylacrylate, 35% BA, 325 MFI, from Exxon;

Brazilian Gum Rosin, from CPM Inc.,

Poly THF, polytetrahydrofuran, obtained from BASF, number indicates approximate molecular weight.

**Example I.** The following compositions were prepared from the above listed raw materials in parts by weight (PBW). Viscosity, STR (dyne/cm), water contact angle ($\Theta$), and heat stability of each composition were tested, and the results are shown.

The water contact angle was measured 5 seconds after dropping with the use of a goniometer, which has a microsyringe for dispensing accurate droplet sizes and a camera for photographing the angle of the liquid drop as it meets the surface of the solid. The water contact angle was measured as the angle between the adhesive and the tangent of the liquid drop (at the interface).

Water surface tension was measured using the Dunuoy ring method. Two grams of each test composition were placed in a clean 110ml glass jar with a 5 cm inner diameter, melted in a 135 degree Celsius oven, and then cooled to room temperature. Twenty ml of 0.85% saline solution was added to the dish. The surface tensions of the pure saline solution and of the saline solution after 15 minutes exposure to the test composition were measured using a KRUS K-14 tensiometer. The difference between the surface tensions was recorded as the STR.

Heat stability was determined after subjecting 30 g of 3 each composition in a 4 oz glass jar covered with aluminum foil to 275 degrees F for 24 hours. Compositions for which no skin formation, gelling, or phase separation occurred were considered to pass the heat stability test.

Composition 1

XW23.AH, 50 PBW
Unitac R-98L, 40 PBW

PPG 3025, 10 PBW
Irganox 1010, 0.5 PBW

Viscosity (cP)@135C: 15,525
STR: 8.1
Water contact angle ($\Theta$): 58
Heat stability: pass

Composition 2

XW23.AH, 50 PBW
XR5108, 40 PBW
PPG 3025, 10 PBW
Irganox 1010, 0.5 PBW

Viscosity (cP)@135C: 15,075
STR: 10.2
Water contact angle ($\Theta$): 42
Heat stability: pass

Composition 3

EVA 18%VA 500MI, 50 PBW
XR 5108, 40 PBW
PPG 3025, 10 PBW
Irganox 1010, 0.5 PBW

Viscosity (cP)@135C: 8,350
STR: 8.5
Water contact angle ($\Theta$): 45
Heat stability: pass

Composition 4

EVA 28%VA 400MI, 50 PBW
Unitac R-98L, 40 PBW
PPG 3025, 10 PBW
Irganox 1010, 0.5 PBW

Viscosity (cP)@135C: 11,575
STR: 12.2
Water contact angle ($\Theta$): 41
Heat stability: pass

Composition 5

EVA 28%VA 400MI, 50 PBW
ECR 179EX, 40 PBW
PPG 3025, 10 PBW
Irganox 1010, 0.5 PBW

Viscosity (cP)@135C: 10,375
STR: 14.8
Water contact angle ($\Theta$): 41
Heat stability: pass

Composition 6

EVA 28%VA 400MI, 50 PBW
ECR 5400, 40 PBW
PPG 3025, 10 PBW
Irganox 1010, 0.5 PBW

Viscosity (cP)@135C: 10,850
STR: 13.9
Water contact angle (Θ): 41
Heat stability: pass

Composition 7

EVA 28%VA 400MI, 50 PBW
Brazilian Gum Rosin, 40 PBW
PPG 3025, 10 PBW
Irganox 1010, 0.5 PBW

Viscosity (cP)@135C: 10,775
STR: 7.6
Water contact angle (Θ): 63
Heat stability: pass

Composition 8

EVA 28%VA 400MI, 10 PBW
XR 5108, 80 PBW
PPG 3025, 10 PBW
Irganox 1010, 0.5 PBW

Viscosity (cP)@135C: 785
STR: 8.0
Water contact angle (Θ): 44
Heat stability: pass

Composition 9

EVA 28%VA 400MI, 80 PBW
XR 5108, 10 PBW
PPG 3025, 10 PBW
Irganox 1010, 0.5 PBW

Viscosity (cP)@135C: 29,300
STR: 14.5
Water contact angle (Θ): 44
Heat stability: pass

Composition 10

EVA 28%VA 400MI, 20 PBW
XR 5108, 70 PBW
PPG 3025, 10 PBW
Irganox 1010, 0.5 PBW

Viscosity (cP)@135C: 2,040
STR: 8.0
Water contact angle (Θ): 45

Heat stability: pass

Composition 11

EVA 28%VA 400MI, 70 PBW
XR 5108, 20 PBW
PPG 3025, 10 PBW
Irganox 1010, 0.5 PBW

Viscosity (cP)@135C: 33,850
STR: 8.6
Water contact angle ($\Theta$): 68
Heat stability: pass

**Example II.** Compositions containing 50% Elvax 140W, 40% Zonatac 105L, 0.5% Irganox 1010, and 10% PPG of various molecular weights were prepared. $\Theta$, the STR, the viscosity, and the heat stability (HS) of each composition was tested, and the results are shown. Viscosity was measured at 135 degrees Celsius, 10 rpm, using a #27 spindle as described in Example I, above. Heat stability (HS) was measured after 24 hours exposure to 135 degrees Celsius as described above. $\Theta$ and STR were also measured as described above.

| PPG MW | $\Theta$ (degree) | STR (dyne/cm) | Viscosity (cP) | HS |
|---|---|---|---|---|
| 425 | 69 | 11.6 | 9,400 | yes |
| 1,000 | 67 | 13.5 | 10,500 | yes |
| 2,000 | 55 | 21.1 | 10,970 | yes |
| 3,000 | 41 | 15.6 | 10,970 | yes |
| 4,025 | 40 | 16.9 | 12,470 | yes |

As can be seen from the above results, compositions containing higher molecular weight PPG had a decreased fluid contact angle, $\Theta$.

**Example III.** Compositions containing 50 parts Elvax 210, 46 parts XR5108, 0.5 parts Irganox 1010, and various concentrations of PPG (3000 MW), were prepared. For each composition, $\Theta$, the STR, the viscosity, and the heat stability (HS) were tested as described above, and the results are shown. Viscosity was measured at 135 degrees Celsius. Heat stability (HS) was measured after 24 hours exposure to 177 degrees Celsius.

| PPG(%wt) | $\Theta$ (degree) | STR (dyne/cm) | Viscosity | HS |
|---|---|---|---|---|
| 1 | 70 | 5.27 | 15,320 | yes |
| 3 | 56 | 7.97 | 11,700 | yes |
| 5 | 48 | 11.8 | 11,800 | yes |

The above results show that increasing the amount of PPG in an EVA copolymer composition lowers the contact angle ($\Theta$) of a fluid, but also reduces the surface tension of the contacting fluid.

**Example IV.** Compositions containing 50 parts Elvax 210, 46 parts XR5108, 0.5 parts Irganox 1010, and increasing amounts of a combination of PPG (3000 MW) and Unithox 480 Ethoxylate ("480"), were prepared. For each composition, $\Theta$, the STR, the viscosity, and the heat stability (HS) were tested as described above, and the results are shown. Viscosity was measured at 135 degrees Celsius. Heat stability (HS) was measured after 24 hours exposure to 177 degrees Celsius temperature. ($\Theta$ is in degrees, and STR is in dyne/cm).

| % wt PG | % wt 480 | $\Theta$ | STR | Viscosity | HS |
|---|---|---|---|---|---|
| 1 | 1 | 61 | 14.2 | 12,600 | yes |
| 3 | 1 | 27 | 14.5 | 11,320 | yes |
| 3 | 3 | 24 | 16.5 | 12,300 | yes |

The results provide an example of how a polyether and a surfactant can be combined to obtain various balances of the properties $\Theta$ and STR.

Example V. Compositions containing 50 parts Elvax 210, 46 parts XR5108, 0.5 parts Irganox 1010, and increasing amounts of Unithox 480 Ethoxylate ("480"), were prepared. For each composition, $\Theta$, the STR, the viscosity, and the heat stability (HS) were tested as described above, and the results are shown. Viscosity was measured at 135 degrees Celsius. Heat stability (HS) was measured after 24 hours exposure to 177 degrees Celsius temperature.

| 480(%wt) | STR $\Theta$ (degree) | (dyne/cm) | Viscosity | HS |
|---|---|---|---|---|
| 0 | 92 | 2.5 | 13,770 | yes |
| 1 | 85 | 1.7 | 13,550 | yes |
| 3 | 72 | 5.9 | 13,420 | top layer darker no separation |
| 5 | 40 | 10.6 | 12,570 | |

A comparison of Examples III, IV, and V, above show that a polyether/surfactant combination achieves a synergistic reduction of the fluid contact angle $\Theta$.

Example VI. Compositions comprising polytetrahydrofuran of various molecular weights were prepared as set forth below. Viscosity, heat stability, and water contact angle ($\Theta$) were measured for each composition as described above, and the results are given. Heat stability was measured after 24 hours at about 135 degrees Celsius (30 g, covered). The water contact angle was measured after 15 seconds, by V.C.. For each composition, the water contact angle shown is an average of 10 measurements, and is plus or minus 3 degrees. A composition (17) comprising the same amount of PPG 3025 was also prepared and tested.

<u>Composition 12</u>

Elvax 210, 50 PBW
ECR-179EX, 40 PBW
PolyTHF 250, 10 PBW
Irganox 1010, 0.5 PBW

Viscosity (cP)@135C: 6,250
Water contact angle ($\Theta$): 76
Heat stability: pass

<u>Composition 13</u>

Elvax 210, 50 PBW
ECR-179EX, 40 PBW
PolyTHF 650, 10 PBW
Irganox 1010, 0.5 PBW

Viscosity (cP)@135C: 10,350
Water contact angle ($\Theta$): 78
Heat stability: pass

Composition 14

Elvax 210, 50 PBW
ECR-179EX, 40 PBW
PolyTHF 1000, 10 PBW
Irganox 1010, 0.5 PBW

Viscosity (cP)@135C: 10,275
Water contact angle ($\Theta$): 77
Heat stability: pass

Composition 15

Elvax 210, 50 PBW
ECR-179EX, 40 PBW
PolyTHF 2000, 10 PBW
Irganox 1010, 0.5 PBW

Viscosity (cP)@135C: 11,575
Water contact angle ($\Theta$): 79
Heat stability: pass

Composition 16

Elvax 210, 50 PBW
ECR-179EX, 40 PBW
PolyTHF 2900, 10 PBW
Irganox 1010, 0.5 PBW

Viscosity (cP)@135C: 11,450
Water contact angle ($\Theta$): 79
Heat stability: pass

Composition 17

Elvax 210, 50 PBW
ECR-179EX, 40 PBW
PPG 3025, 10 PBW
Irganox 1010, 0.5 PBW

Viscosity (cP)@135C: 11,100
Water contact angle ($\Theta$): 45
Heat stability: pass

**Example VII.** The following compositions comprising Nuwet 500 were prepared according to the subject invention, and contact angle, STR, and viscosity were tested as above; liquid strike through was determined according to the EDANA 150.2.93 method:

Composition 18

EVA 33%VA 400MI, 50 PBW
ECR 179EX, 40 PBW
Nuwet 500, 10 PBW
Irganox 1010, 0.5 PBW

$\Theta$: 25
STR: 40 (dyne/cm)
Liquid Strike Through (seconds): 5

Viscosity @ 130 degrees C: 19,600
Viscosity @ 160 degrees C: 6,800

Composition 19

EVA 28%VA 400MI, 50 PBW
ECR 179EX, 40 PBW
Nuwet 500, 3 PBW
PPG MW3000, 7 PBW
Irganox 1010, 0.5 PBW
Θ: 16
STR: 29 (dyne/cm)
Liquid Strike Through (seconds): 4
Viscosity @ 130 degrees C: 15,100
Viscosity @ 160 degrees C: 5,900

**Example VIII.** The above composition 19 as well as compositions containing Nuwet 550 and Aerosol OT100 were tested for repeat strike through time:

Composition 19

Viscosity @ 130 degrees C: 16,800
Liquid Strike Through (seconds): 5.63

Dose 1    : 6.95
Dose 2:    6.65
Dose 3:    5.95
Dose 4:    5.52
Dose 5:    6.18

Wetback (grams): 0.34
Liquid Contact Angle (@ 5 seconds): wets out

Composition 20

EVA 28%VA 400MI, 50 PBW
ECR 179EX, 40 PBW
Aerosol OT100, 3 PBW
PPG MW3000, 7 PBW
Irganox 1010, 0.5 PBW

Viscosity @ 130 degrees C: 14,000
Liquid Strike Through (seconds): 6.29

Dose 1:    6.58
Dose 2:    6.10
Dose 3:    6.10
Dose 4:    6.51
Dose 5:    6.03

Wetback (grams): 0.34
Liquid Contact Angle (@ 5 seconds): 33

Composition 21

EVA 28%VA 400MI, 50 PBW
ECR 179EX, 40 PBW
Nuwet 550, 3 PBW

PPG MW3000, 7 PBW

Irganox 1010, 0.5 PBW

Viscosity @ 130 degrees C: 12,900

Liquid Strike Through (seconds): 7.42

Dose 1:     7.61
Dose 2:     7.30
Dose 3:     7.30
Dose 4:     5.83
Dose 5:     6.43

Wetback (grams): 0.20

Liquid Contact Angle (@ 5 seconds): wets out

The hydrophilic nonwoven (a nonwoven treated with a hydrophilic finish) and hydrophilic adhesive (composition 19) laminated onto a hydrophobic nonwoven (6g slot coating) give similar strike through times of 2-3 seconds initially. However, when subjected to repeat doses, the hydrophilic nonwoven gives increasingly longer strike through times. This is due to the leaching out of surfactant from the nonwoven surface and hence the nonwoven loses its hydrophilic properties giving final strike through values at five doses of approximately 42 seconds. The hydrophilic hot melt (composition 19) retains its hydrophilic nature after repeat strike through giving continuous low values of about 4 to 6 seconds.

Further embodiments of the present invention include:

Embodiment 1 comprises an absorbent article comprising a hot melt adhesive composition, which composition comprises:

a) a base polymer in an amount effective to render the composition adhesive; and
b) a mixture of one or more polyethers in an amount effective to cause a fluid contacting the article to exhibit a fluid contact angle of less than about 90 degrees and a fluid surface tension reduction of less than about 40 dyne/cm, said polyethers consisting essentially of homo, alternating, or random units having the formula

-R-O-,

wherein each R is selected from the group consisting of alkyl moieties having 2 or more carbon atoms.

Embodiment 2 comprises an absorbent article according to embodiment 1, wherein each R is selected from the group consisting of alkyl moieties having from 2 to 5 carbon atoms.

Embodiment3 comprises an absorbent article according to embodiment 2, wherein the mixture comprises at least one polyether which includes units wherein R has 3 carbon atoms.

Embodiment4 comprises an absorbent article according to embodiment 3, wherein the mixture comprises polypropyleneglycol.

Embodiment5 comprises an absorbent article according to embodiment 2, wherein the mixture comprises at least one polyether which includes units wherein R has 4 carbon atoms.

Embodiment6 comprises an absorbent article according to embodiment 5, wherein the mixture comprises polytetrahydrofuran.

Emhodiment7 comprises an absorbent article according to embodiment 1, wherein the polyethers of the mixture have molecular weights of about 425 Daltons or higher.

Embodiment8 comprises an absorbent article according to embodiment 7, wherein the polyethers of the mixture have molecular weights between about 2000 Daltons and about 4000 Daltons.

Embodiment9 comprises an absorbent article according to embodiment 4, wherein the polypropyleneglycol has a molecular weight between about 2000 Daltons and about 4000 Daltons.

Embodiment10 comprises an absorbent article according to embodiment 9, wherein the polypropyleneglycol has a molecular weight of about 3000 Daltons.

Embodiment11 comprises an absorbent article according to embodiment 1, wherein the hot melt adhesive composition bonds at least two components of the article together.

Embodiment12 comprises an absorbent article according to embodiment 1, which article comprises at least one component comprised of a nonwoven fabric, and wherein the hot melt adhesive composition bonds together fibers of said nonwoven fabric.

Embodiment13 comprises an absorbent article according to embodiment 1, wherein the hot melt adhesive composition further comprises an effective amount of one or more additives selected from the group consisting of tackifying resins, plasticizers, stabilizers, and waxes.

Embodiment14 comprises an absorbent article according to embodiment 1, wherein the hot melt adhesive composition comprises from about 10 to about 80 percent by weight of the base polymer of (a); and from about 1 to about 25 percent by weight of the mixture of (b).

Embodiment15 comprises an absorbent article according to embodiment 14, wherein the hot melt adhesive composition comprises from about 40 to about 60 percent by weight of the base polymer.

Embodiment16 comprises an absorbent article according to embodiment 14, wherein the hot melt adhesive composition comprises from about 3 to about 10 percent by weight of the mixture (b).

Embodiment17 comprises an absorbent article comprising a hot melt adhesive composition, which composition comprises:

a) a base polymer in an amount effective to render the composition adhesive;
b) a mixture of one or more polyethers consisting essentially of homo, alternating, or random units having the formula

$$-R-O-,$$

wherein each R is selected from the group consisting of alkyl moieties having 2 or more carbon atoms; and
c) one or more surfactants;

said mixture and said surfactants being present in amounts which in combination are effective to cause a fluid contacting the article to exhibit a fluid contact angle of less than about 90 degrees and a fluid surface tension reduction of less than about 40 dyne/cm.

Embodiment18 comprises an absorbent article according to embodiment 17, wherein each R is selected from the group consisting of alkyl moieties having from 2 to 5 carbon atoms.

Embodiment19 comprises an absorbent article according to embodiment 18, wherein the mixture comprises at least one polyether which includes units which have 3 carbon atoms.

Embodiment20 comprises an absorbent article according to embodiment 19, wherein the mixture comprises polypropyleneglycol.

Embodiment21 comprises an absorbent article according to embodiment 18, wherein the mixture comprises at least one polyether which include units which have 4 carbon atoms.

Embodiment22 comprises an absorbent article according to embodiment 21, wherein the mixture comprises polytetrahydrofuran.

Embodiment23 comprises an absorbent article according to embodiment 17, wherein the polyethers of the mixture have molecular weights of about 425 Daltons or higher.

Embodiment24 comprises an absorbent article according to embodiment 23, wherein the polyethers of the mixture have molecular weights between about 2000 Daltons and about 4000 Daltons.

Embodiment25 comprises an absorbent article according to embodiment 20, wherein the polypropyleneglycol has a molecular weight between about 2000 Daltons and about 4000 Daltons.

Embodiment26 comprises an absorbent article according to embodiment 25, wherein the polypropyleneglycol has a molecular weight of about 3000 Daltons.

Embodiment27 comprises an absorbent article according to embodiment 17, wherein the amounts of the mixture and of the surfactant or surfactants are in combination effective to cause a fluid contacting the article to exhibit a fluid contact angle of less than about 50 degrees.

Embodiment28 comprises an absorbent article according to embodiment 27, wherein the amounts of the mixture and of the surfactant or surfactants are in combination effective to cause a fluid contacting the article to exhibit a fluid contact angle of less than about 25 degrees.

Embodiment29 comprises an absorbent article according to embodiment 17, wherein (c) comprises a surfactant selected from a nonionic surfactant, an anionic surfactant, or a cationic surfactant.

Embodiment30 comprises an absorbent article according to embodiment 29, wherein (c) comprises a nonionic surfactant, and the nonionic surfactant is a silicone surfactant.

Embodiment31 comprises an absorbent article according to embodiment 17, comprising Nuwet 500.

Embodiment32 comprises an absorbent article according to embodiment 17, wherein (c) comprises Nuwet 550.

Embodiment33 comprises an absorbent article according to embodiment 29, wherein (c) comprises a nonionic surfactant, and the nonionic surfactant is a linear alcohol.

Embodiment34 comprises an absorbent article according to embodiment 33, wherein the linear alcohol is Unithox 480 Ethoxylate.

Embodiment35 comprises an absorbent article according to embodiment 29, wherein (c) comprises an anionic surfactant, and the anionic surfactant is a dialkyl sulfosuccinate.

Embodiment36 comprises an absorbent article according to embodiment 17, wherein the hot melt adhesive composition bonds at least two components of the article together.

Embodiment37 comprises an absorbent article according to embodiment 17, which article comprises at least one component comprised of a nonwoven fabric, and wherein the hot melt adhesive composition bonds together fibers of said nonwoven fabric.

Embodiment38 comprises an absorbent article according to embodiment 17, wherein the hot melt adhesive composition further comprises an effective amount of one or more additives selected from the group consisting of tackifying resins, plasticizers, stabilizers, and waxes.

Embodiment39 comprises a hot melt adhesive composition, which composition comprises:

a) a base polymer in an amount effective to render the composition adhesive;
b) a mixture of one or more polyethers consisting essentially of homo, alternating, or random units having the formula

-R-O-,

wherein each R is selected from the group consisting of alkyl moieties having 2 or more carbon atoms; and
c) one or more surfactants;

said mixture and said surfactants being present in amounts which in combination are effective to cause a fluid contacting the hot melt adhesive composition to exhibit a fluid contact angle of less than about 90 degrees and a fluid surface tension reduction of less than about 40 dyne/cm.

Embodiment40 comprises an absorbent article comprising a hot melt adhesive composition comprising from about 10 to about 80 percent by weight of an ethylene/vinyl acetate copolymer, from about 3 to about 10 percent by weight of polypropyleneglycol, and from about 0.5 to about 10 percent by weight of Nuwet 500 or Nuwet 550.

Embodiment41 comprises an absorbent article comprising a hot melt adhesive composition comprising from about 10 to about 80 percent by weight of an ethylene/vinyl acetate copolymer and from about 3 to about 10 percent by weight of Nuwet 500.

## Claims

1. An absorbent article comprising a hot melt adhesive composition, which composition comprises:

   a) a base polymer in an amount effective to render the composition adhesive; and
   b) a mixture of one or more polyethers in an amount effective to cause a fluid contacting the article to exhibit a fluid contact angle of less than about 90 degrees and a fluid surface tension reduction of less than about 40 dyne/cm, said polyethers consisting essentially of homo, alternating, or random units having the formula

   -R-O-,

   wherein each R is selected from the group consisting of alkyl moieties having 2 or more carbon atoms.

2. An absorbent article according to claim 1 or 17 wherein each R is selected from the group consisting of alkyl moieties having from 2 to 5 carbon atoms.

3. An absorbent article according to claim 2 wherein the mixture comprises polypropyleneglycol.

4. An absorbent article according to claim 2 wherein the mixture comprises polytetrahydrofuran.

5. An absorbent article according to claim 1 or 11 wherein the polyethers of the mixture have molecular weights of about 425 Daltons or higher.

6. An absorbent article according to claim 5 wherein the polyethers of the mixture have molecular weights between about 2000 Daltons and about 4000 Daltons.

7. An absorbent article according to claim 1 or 11 wherein the hot melt adhesive composition bonds at least two components of the article together.

8. An absorbent article according to claim 1 or 11 which article comprises at least one component comprised of a nonwoven fabric, and wherein the hot melt adhesive composition bonds together fibers of said nonwoven fabric.

9. An absorbent article according to claim 1 or 11 wherein the hot melt adhesive composition further comprises an effective amount of one or more additives selected from the group consisting of tackifying resins, plasticizers, stabilizers, and waxes.

10. An absorbent article according to claim 1, wherein the hot melt adhesive composition comprises from about 10 to about 80 percent by weight of the base polymer of (a); and from about 1 to about 25 percent by weight of the mixture of (b).

11. An absorbent article comprising a hot melt adhesive composition, which composition comprises:

   a) a base polymer in an amount effective to render the composition adhesive;
   b) a mixture of one or more polyethers consisting essentially of homo, alternating, or random units having the formula

$$-R-O-,$$

   wherein each R is selected from the group consisting of alkyl moieties having 2 or more carbon atoms; and
   c) one or more surfactants;

said mixture and said surfactants being present in amounts which in combination are effective to cause a fluid contacting the article to exhibit a fluid contact angle of less than about 90 degrees and a fluid surface tension reduction of less than about 40 dyne/cm.

12. An absorbent article according to claim 11, comprising Nuwet 500.

13. An absorbent article according to claim 11, wherein (c) comprises Nuwet 550.

14. A hot melt adhesive composition, which composition comprises:

   a) a base polymer in an amount effective to render the composition adhesive;
   b) a mixture of one or more polyethers consisting essentially of homo, alternating, or random units having the formula

$$-R-O-,$$

   wherein each R is selected from the group consisting of alkyl moieties having 2 or more carbon atoms; and
   c) one or more surfactants;

said mixture and said surfactants being present in amounts which in combination are effective to cause a fluid contacting the hot melt adhesive composition to exhibit a fluid contact angle of less than about 90 degrees and a fluid surface tension reduction of less than about 40 dyne/cm.

15. An absorbent article comprising a hot melt adhesive composition comprising from about 10 to about 80 percent by weight of an ethylene/vinyl acetate copolymer, from about 3 to about 10 percent by weight of polypropyleneglycol, and from about 0.5 to about 10 percent by weight of Nuwet 500 or Nuwet 550.